# EUROPEAN PATENT APPLICATION

(11) **EP 2 347 761 A1**
(43) Date of publication of application: **27.07.2011**
(21) Application number: 09814081.7
(22) Date of filing: 22.09.2009
(51) Int. Cl.: A61K 38/19, A61P 35/00

(54) **A COMPOSITION WITH INHIBITION ACTIVITY ON PATHOLOGICAL ANGIOGENESIS**

(30) Priority: 22.09.2008 US 136635 P
(71) Applicant: TTY Biopharm Company Limited, Taipei, Taiwan (TW)
(72) Inventor: KUO, Min-Liang, Taipei City, Taiwan (CN); WU, Yu-Ling, Taipei City Taiwan (CN)
(74) Representative: Trösch, Hans-Ludwig
(86) International application number: PCT/CN2009/074086
(87) International publication number: WO 2010/031361

(57) **Abstract**

The present invention provides a pharmaceutical composition for inhibiting pathologic angiogenesis and/or cell proliferative disorder. The pharmaceutical composition of the present invention comprises an effective amount of LECT2 protein or analogue thereof, and a pharmaceutically acceptable carrier.

## Description

### Background of the Invention

### Field of the Invention

The present invention relates to a method for inhibiting angiogenesis, and particularly relates to inhibition of angiogenesis and/or angiogenesis-associated diseases with LECT2 protein.

### Description of the Related Art

Leukocyte cell-derived chemotaxin 2 (hereinafter LECT2), a 16-kDa protein, is a novel neutrophil chemotactic factor which has already been purified from the culture of human T-cell leukemia SWK3 cell induced by phytohemagglutinin (Biochim Biophys Acta 1396:105-13 (1998)). In addition, its mRNA has been known to specifically express in adult or fetal liver and human liver cancer cell strains by isolation of cDNA that encodes LECT2 (Biochem Biophys Res Commun 237:116-20 (1997)). LECT2 is a chondromodulin-II (a cartilage regulatory factor) which is a bovine protein stimulating the proliferation of chondrocytes and osteoblasts (J Bio Chem 125:436-42 (1999)). Currently, it has been reported that the expression of murine LECT2 is transiently reduced in hepatitis induced by concanavaline A (Con A). Such hepatitis induced by Con A is an animal model of human autoimmune hepatitis that can be induced by immunocyte (such as CD4+ T cells or macrophages) associated cytohormones or cytotoxic molecules (J Clin Invest 90:196 (1992)). To date, however, it has not been revealed in any literature that LECT2 protein can inhibit angiogenesis.

Angiogenesis refers to the generation of new vessels to repair injuries and resume the blood supply of tissues, which occurs when a healthy individual suffers injuries. The process of angiogenesis is strictly regulated by various positive and negative regulatory factors. However, in many disease conditions, the angiogenesis of patients is out of control.

Angiogenesis is an important process to the growth of newborns, and is also very important to injury repair. It, however, is also a pathologic feature of many clinical diseases including tissue inflammation, arthritis, tumor growth, diabetic retinopathy, degeneration of macula caused by retinal angiogenesis, and the like. These clinical manifestations associated with angiogenesis are also known as angiogenic diseases. Angiogenic diseases generally include cancer (including solid and liquid tumors), cardiovascular diseases (such as arteriosclerosis), chronic inflammation (such as rheumatoid arthritis and Crohn's disease), diabetes (such as diabetic retinopathy), psoriasis, endometriosis and obesity.

Therefore, there is an urgent need in medical field for a method and composition to effectively inhibit angiogenesis to treat or prevent the aforementioned diseases.

### Summary of the Invention

The present invention provides a method for inhibiting pathologic angiogenesis, comprising administering an effective amount of LECT2 protein or analogue thereof, and a pharmaceutically acceptable carrier to an individual.

The present invention also provides a composition that inhibits angiogenesis, comprising an effective amount of LECT2 protein or analogue thereof, and a pharmaceutically acceptable carrier.

The present invention further provides a method for treatment and/or inhibition of cell proliferative disorders, comprising administering an effective amount of LECT2 protein or analogue thereof, and a pharmaceutically acceptable carrier to an individual.

The present invention further provides a composition that treats and/or inhibits cell proliferative disorders, comprising an effective amount of LECT2 protein or analogue thereof, and a pharmaceutically acceptable carrier.

The present invention further provides a method for inhibiting pathologic angiogenesis, comprising administering an effective amount of LECT2 protein or analogue thereof, and a pharmaceutically acceptable carrier to an individual.

In order to render such and other purposes, aspects, and advantages of the present invention obvious and clear, preferred embodiments are specified in the following and illustrated below in detail in conjunction with the attached figures.

### Brief Description of the Figures

Figure 1A shows the immunohistochemical staining analysis of human liver cancer microvascular density (CD34 staining).
Figure 1B shows the correlation between LECT2 protein expression level and HCC microvascular density.
Figure 2A shows that the growth curves of the vector control cells (SK-Hep1/Neo), LECT2-overexpressing cells (SK-Hep1/LECT2) and wild-type cells (SK-Hep1/Wn are similar.
Figure 2B shows that the average tumor volume of SK-Hep1/Neo group is significantly larger than that of SK-Hep1/LECT2 group.
Figure 2C shows that the microvascular density of SK-Hep1/LECT2 group (CD31 staining) is smaller than vector control group (SK-Hep1/Neo).
Figure 3A shows that the growth curves of the vector control cells (BNL/Neo), LECT2-overexpressing cells (BNULECT2) and wild-type cells (BNL/WT) are similar.
Figure 3B shows that the average tumor volume of BNL/Neo group is significantly larger than that of BNULECT2 group.
Figure 3C shows that LECT2 inhibits the microvascular density in BNL cell tumor *in situ* of BNL cells.
Figure 4A shows the tumor volumes of BNL/Neo group, BNL/Neo+rLECT2 group and BNULECT2 group, respectively.
Figures 4B-4C show that the average tumor volume of BNL/Neo group is significantly larger than those of BNL/Neo+rLECT2 group and BNULECT2 group.
Figure 5 shows that the conditioned media of LECT2-overexpressing cells can disrupt angiogenesis.
Figure 6A shows that LECT2 conditioned media can inhibit formation of vascular tube-like structures.
Figure 6B shows the expression level of LECT2 in HCC transplant strains (PLC/PRF/5 and Huh-7) in which endogenous LECT2 (shLECT2-1 and shLECT2-2) is suppressed.
Figure 6C shows that compared with control cells, the conditioned media of PLC/PRF/5 and Huh-7 cells in which endogenous LECT2 is suppressed can improve the formation of vascular tube-like structures in vascular endothelial cells.
Figure 7 shows that LECT2 conditioned media significantly inhibit the migration of vascular endothelial cells.
Figure 8 shows that compared with cells treated with VEGF-A (50 ng/mL), rLECT2 protein can significantly inhibit VEGF-A induced formation of tube-like structures.
Figure 9A-9B show that LECT2 recombinant protein is confirmed to inhibit VEGF-A induced angiogenesis by CAM model.
Figure 10 shows that LECT2 recombinant protein can reduce VEGF-A induced migration of vascular endothelial cells.
Figure 11 shows that LECT2 recombinant protein can inhibit VEGF-A induced formation of vascular tube-like structures in vascular endothelial cells.
Figure 12 shows that LECT2 recombinant protein can inhibit formation of vascular tube-like structure induced by conditioned media in vascular endothelial cells.
Figures 13A-13B show that the average tumor volume of B16F1/Neo group is significantly larger than that of B16F1/LECT2 group.
Figure 13C shows that the microvascular density of B16F1/LECT2 group is smaller than that of B16F1/Neo group.
Figure 14 shows that LECT2 protein can inhibit the tumor growth of A549 cells in nude mice.

### Detailed Description of the Invention

The present invention provides a composition that inhibits pathologic angiogenesis and/or cell proliferative disorders. Such composition comprises an effective amount of LECT2 protein and analogue thereof, and a pharmaceutically acceptable carrier.

"LECT2" or "LECT2 protein" as described herein refers to all forms of leukocyte-derived chemotaxin 2 protein including active or inactive forms. Any analogue/fragment of LECT2 protein that has biological activity is within the scope of the present invention. Analogue/fragment can be conjugated to carrier molecules such as immunoproteins for various applications, including increasing valence of protein binding sites. Furthermore, the LECT2 protein analogue of the present invention can also be obtained by replacement, deletion, addition or insertion of one or more amino acids in the original protein and still retains at least one active component of the original protein.

"LECT2 mutants" or "mutant proteins" as described herein include proteins that share at least 60% sequence identity with LECT2 protein, wherein sequence similarity is defined by the amino acid sequences of the proteins to maximize the common components and minimize sequence difference.

The LECT2 proteins of the present invention also include modified LECT2 proteins which are analogous to the native LECT2 protein. A person skilled in the art can modify peptide or DNA sequences with common and conventional techniques. For example, modification of protein sequences may include alteration, replacement, substitution, insertion, or deletion of one amino acid in the coding sequence. The methods employed in alteration, replacement, substitution, insertion, or deletion of the sequences are commonly known techniques. The sequences preferably retain the activity of LECT2 protein after the alteration, replacement, substitution, insertion or deletion.

"A recombinant protein" as described herein refers to a protein comprising one or more recombinant components. Site-specific recombinases are a group of enzymes existing in various viruses and bacteria, which have both endonuclease and ligase activity. Such recombinases can recognize specific DNA sequences and alter DNA fragments.

In the present invention, LECT2 protein, LECT2 protein analogues/fragments or recombinant LECT2 proteins can be expressed in animal cells, low eukaryotes or prokaryotes. Animal cells may include monkey COS cells, CHO cells, human renal 293 cells, human epidermal A431 cells, human Colo205 cells, 3T3 cells, CV-1 cells, other transformed primate cells, normal diploid cells, *in vitro* cultured primary tissue-derived cell strains, primary colonies, Hela cells, murine L cells, BHK, HL-60, U937, HaK or Jurkat cells. Low eukaryotes include yeast, such as *Saccharomyces cerevisiae, Schizosaccharomyces pombe, Kluyveromyces* strains, *Candida* or any yeast that can express heterogeneous proteins. Prokaryotes may include *Escherichia coli, Bacillus subtilis, Salmonella typhimurium,* or any bacterium that can express heterogeneous proteins. If yeasts or bacteria are used to express a protein, the expressed protein can be further modified, for example, by phosphorylation or glycosylation at approrpiate sites to impart function to the protein.

In the present invention, a composition is applied to a tissue having pathologic angiogenesis, wherein the composition comprises an effective amount of LECT2 protein or a nucleotide vector expressing active LECT2 protein. The tissue can be any tissue or organ undergoing angiogenesis, including brain, skin, muscle, stomach, connective tissues, joints, bones, and the like. Pathologic angiogenesis can be induced by a growth factor, including vascular endothelial growth factor (VEGF), platelet-derived growth factor (PDGF), fibroblast growth factor (FGF), epidermal growth factor (EGF), and hepatocyte growth factor (HGF).

In addition, the LECT2 protein, LECT2 protein analogues/fragments or LECT2 recombinant proteins can also inhibit the generation of tube-like structures of nascent vessels, migration and proliferation of endothelial cells, vascular invasion of tumors, or other angiogenesis-associated diseases. Angiogenesis-associated diseases include, but are not limited to, inflammatory diseases, chronic rheumatoid arthritis and psoriasis, diseases associated with abnormal vascular invasion, for example, diabetic retinopathy, neovascular glaucoma, restenosis of vessels, and cell proliferative disorders, such as tumor or tumor-associated diseases (for example, solid tumors, solid tumor metastasia, liver cancer, lung cancer, breast cancer, colorectal cancer, stomach cancer and melanoma).

"Cell proliferative disorders" as described herein refer to damages to a multiple-cell tissue caused by abnormal proliferation of one or more cell populations in the multiple-cell tissue. Cell proliferative disorders can occur in different animals and in human. Cell proliferative disorders include, but are not limited to, cancer, vessel hyperplasia and fibrosis.

The LECT2 protein and/or the pharmaceutical composition comprising LECT2 protein of the present invention can be administered orally, parenterally, by inhalation, rectally, vaginally, intradermally, transdermally or topically. A dosage unit can include a conventional nontoxic carrier, adjuvant or vehicle that is pharmaceutically acceptable, and can be administered by peristaltic means.

The LECT2 protein and/or the pharmaceutical composition comprising LECT2 protein of the present invention can be administered once, administered multiple times within 24 hours or administered consecutively. When the injection is consecutively administered, an appropriate well-known way can be selected, including but not limited to, intravenous infusion, intravenous injection pump, imbedded injection pump or topical administration. The time of treatment can be properly adjusted according to different circumstances, for example, the progression and severity of angiogenesis. LECT2 protein is administered alone or combined with other agents of the present invention until the termination of pruritus or the treatment is throughout the lifetime.

In another example, the present invention provides a pharmaceutical composition that inhibits pathologic angiogenesis and/or cell proliferative disorder. Such pharmaceutical composition comprises an effective amount of LECT2 protein or analogue thereof, and a pharmaceutically acceptable carrier. Pharmaceutically acceptable carriers include solvents, dispering agents, coatings, antibiotics/antifungal agents, isotonic agents, absorption delaying agents and the like.

In another example, the present invention further provides a method for inhibiting pathologic angiogenesis, including administering an effective amount of LECT2 protein or analogue thereof, and a pharmaceutically acceptable carrier to an individual.

### Examples

### Example 1: The effect of LECT2 protein on human microvascular density

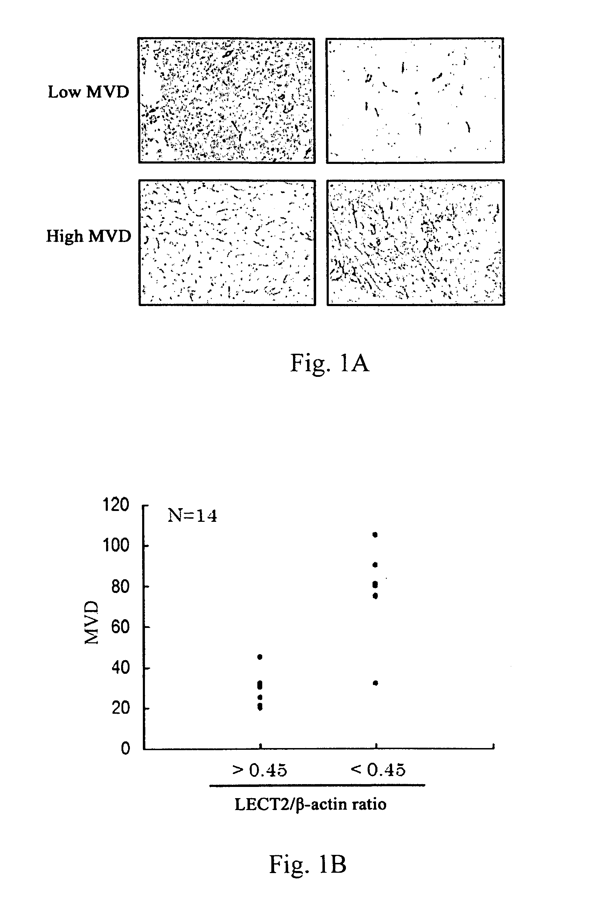

First, the biomarkers of endothelial cells and the expression of LECT2 protein in 14 human liver cancer (HCC) tissues provided by National Taiwan University Hospital were analyzed by immunohistochemical staining. Briefly, primary liver cancer tissue were obtained from patients having liver cancer, fixed with formalin and embedded in paraffin, followed by immunostaining. After deparaffinage and dehydration treatment, they are washed 3 times with PBST and treated with 1:500 dilution of CD34 antibody followed by biotin-labeled anti-mouse IgG secondary antibody (Vector Laboratories, Inc., Burlingame, CA) for 1 hour at room temperature, and the bound antibody was probed with ABC kit (Vector Laboratories Inc.). The sections were stained with diaminobenzidine, washed, and finally counterstained with Delafield's hematoxylin before analysis and counting. Microvascular density and extent of angiogenesis were assayed by staining the endothelial cell antigen. The measurement of microvascular density (MVD) was presented as the count of CD34 antibody-stained endothelial cells. For each tumor, three highly-vascularized regions were analyzed in high magnitude. Count the total number of microvessels in each region, and the average number of microvessels in each tumor. Figure 1A shows the analysis result of human HCC microvascular density, in which the positions of tumors were stained by CD34 antibody. In all tumor samples, the CD34 marker of endothelial cells of liver tumors assayed by immunohistochemical staining had confirmed that many endothelial cells existed surrounding the tumor cells, which was consistent with the clinical discovery of highly-vascularized tumors. According to Figure 1B, the expression level of LECT2 protein shows an inverse tendency to the microvascular density. The liver cancer cells of the patient were divided into two groups, including those with high LECT2 protein expression level (LECT2/β-actin ratio > 0.45) and those with low LECT2 protein expression level (LECT2/β-actin ratio < 0.45). Statistically, high LECT2 protein expression level highly correlated with low microvascular density. Therefore, in the 14 human liver cancer samples, the expression level of LECT2 protein shows an inverse tendency to the microvascular density.

### Example 2: The effect of LECT2 protein on tumor growth of nude mice

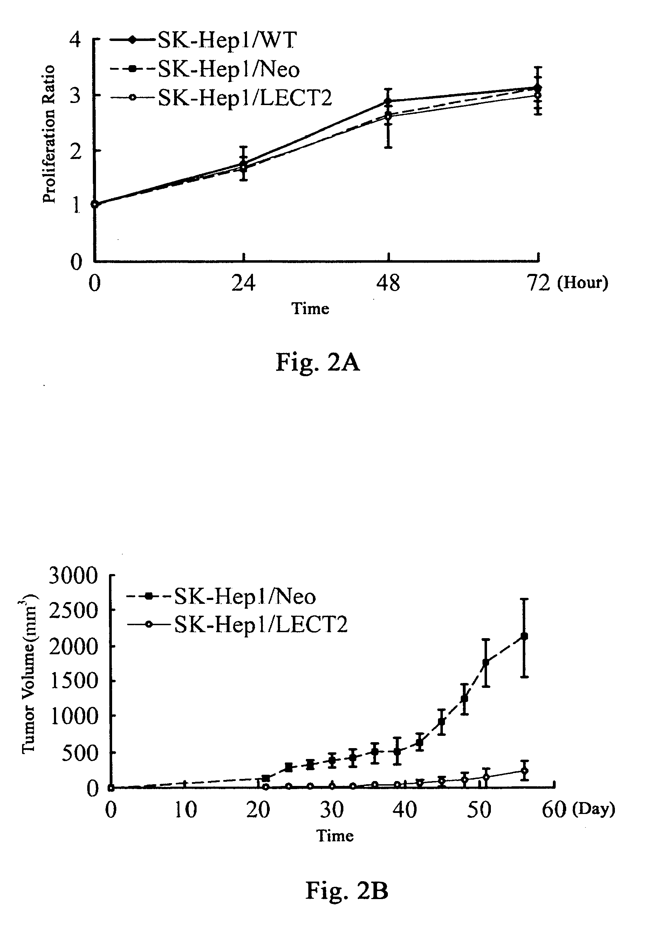

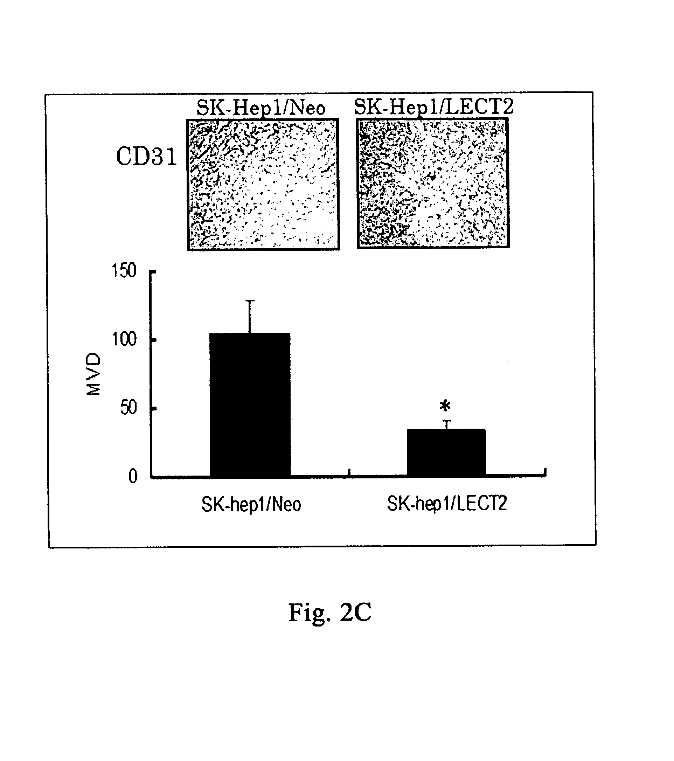

In this example, SK-Hep1, a human liver cancer cell strain without endogenous expression of LECT2 protein was used. cDNA of human LECT2 was constructed into pSecTag2A eukaryotic expression vector to obtain an expression vector overexpressing LECT2 protein and having hygromycin B gene downstream to the same promoter. The LECT2 protein expression vector was used to transform liver cancer cell (SK-Hep1) with Lipofectamine 2000 reagent (Invitrogen Life tech.). After screening with hygromycin B, a cell strain overexpressing LECT2 protein (SK-Hep1/LECT2) was obtained. The expression level of LECT2 protein in the LECT2-overexpressing cell strain (SK-Hep1/LECT2) was at least 5 times higher than that in control cell strain (SK-Hep1/Neo). From Figure 2A, control cell strain (SK-Hep1/Neo), wild-type cell strain (SK-Hep1/WT) and LECT2-overexpressing cells train (SK-Hep1/LECT2) shared similar growth curve of single layer cultivated cell. All experiments of this example were performed in triplicate. Error bars represented 95% confidential interval. To analyze the effect of LECT2 protein on tumor cells and angiogenesis, HCC cells were subcutaneously injected into backs of nude mice, employing an *in vivo* heterologous tumor implantation model. Briefly, 8-week old female thymectomized nude mice (purchased from the National Animal Center) were raised in an environment free of pathogens, and continuously fed and provided with water. Nude mice were randomly divided into two groups, including those injected with LECT2 protein overexpressing cell strain (SK-Hep1/LECT2) and those injected with control cell strain (SK-Hep1/Neo), respectively. At Day 10 after injection, when tumors became obviously visible, tumor sizes were measured every other day. The volume of tumor was presented as wide x length x 0.5. The tumor sizes were measured with caliper. Obvious tumors were first measured 10 days after injection of cells for all mice. According to Figure 2B, at Day 20 after injection of cells, the average tumor volume of nude mice injected with SK-Hep1/Neo cell strain was significantly larger than the average tumor volume of nude mice injected with SK-Hep1/LECT2 cell strain. In addition, the effect of LECT2 on microvascular density in the paraffin sections of *in situ* tumor tissues was further analyzed with CD31 antibody using immunohistochemical staining. From Figure 2C, it can be known that tumor generated from cells stably and highly expressing LECT2 had 3 times less microvessel number per mm² unit volume compared with the control group. From this example, it is known that LECT2 protein inhibits the growth of SK-Hep1 cells of human hepatocarcinoma tumor in nude mice.

### Example 3: The effect of LECT2 protein on tumor growth of BALB/c mice

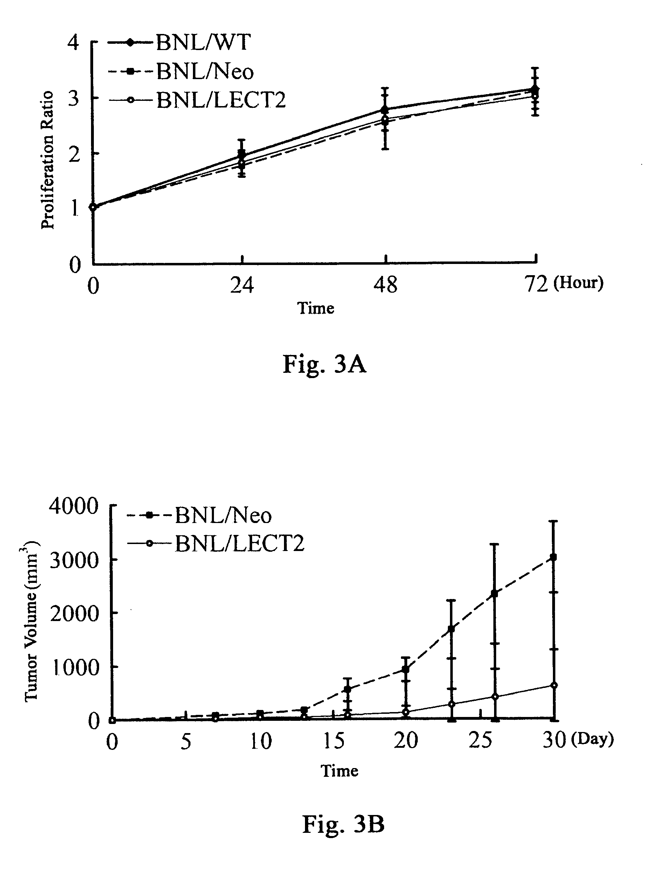

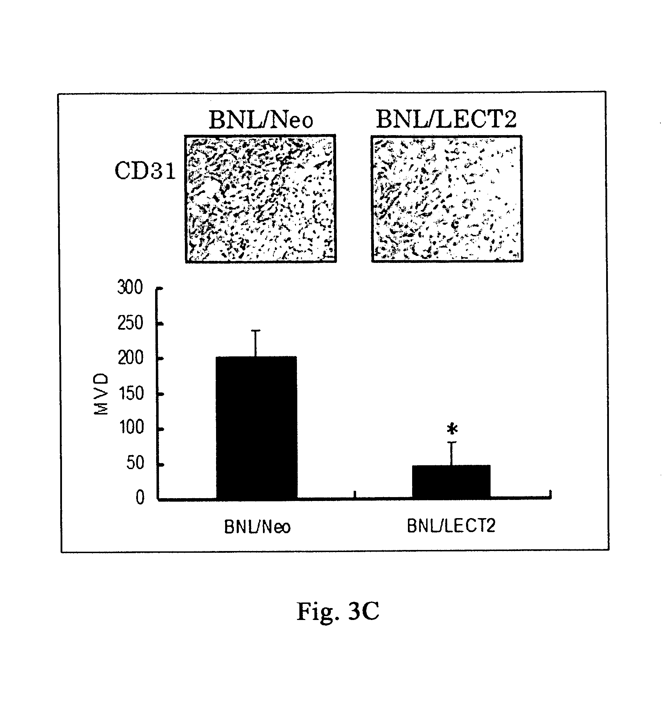

Furthermore, the tumor cells overexpressing LECT2 protein were injected into BALB/c mice and analysis was conducted. Other than replacement of SK-Hep1 human liver cancer cells and nude mice with BNL murine liver cancer cells and BALB/c mice, the analysis of this example was the same as Example 2. Mice (BALB/c mice, 8-week old) were subcutaneously injected with BNL murine liver cancer cells stably and highly expressing LECT2 (BNULECT2) or control BNL cells (BNUNeo), with each mouse injected with 1x10⁵ cells. The results of this example were similar to those of Example 2. According to Figure 3A, wild-type cell strain (BNL/WT, control cell strain (BNL/Neo) and LECT2 protein overexpressing cell strain (BNULECT2) all shared similar growth curves of single layer cultivated cells. According to Figure 3B, the tumor volume of mice injected with BNL/Neo cell strain was significantly larger than the tumor volume of mice injected with BNULECT2 cell strain, and it was found after analysis of microvascular density in *in situ* tumor tissues with CD31 antibody that the microvascular density could be inhibited by high LECT protein expression (Figure 3C). The results of this example show that LECT2 protein inhibits the growth of BNL cells of murine hepatocarcinoma tumor in BALB/c mice.

### Example 4: The effect of the LECT2 recombinant protein on tumor growth of BALB/c mice

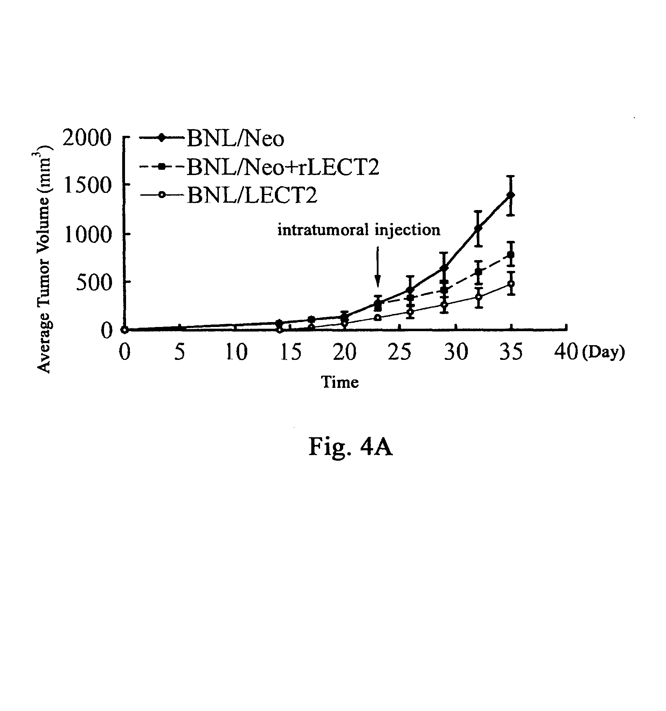

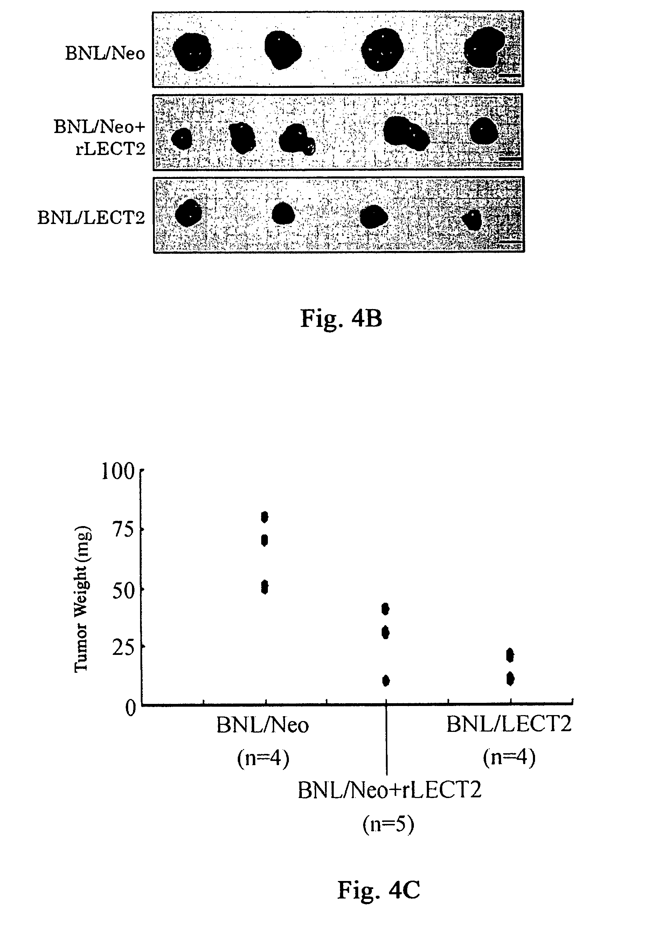

This example analyzed whether the LECT2 recombinant protein could similarly affect the mice transplanted with HCC tumor and assessed the effect of LECT2 recombinant protein on BALB/c mice injected with BNL/Neo cells. When the tumors of the BNL/Neo group grew to about 150 mm³, the LECT2 recombinant protein (1.25 mg/kg body weight) or 1 X PBS buffer (50 µL), was injected into the tumors consecutively for 10 days, respectively. The results were shown in Figure 4A. According to Figure 4B-4C, 35 days after injection with the cells, the tumors of BNL/Neo group had average volumes significantly larger than those injected with the BNULECT2 group which highly expressed LECT2 and the BNULECT2+rLECT2 group which was injected with the LECT2 recombinant protein. Figure 4C showed the tumor weight of each group of BALB/c mice transplanted with heterogeneous tumor. Similarly, the tumor weight of BNULECT2+rLECT2 group was significantly smaller than that of BNL/Neo group. Therefore, LECT2 recombinant protein can inhibit the growth of mouse liver hepatocarcinoma tumor BNL cells in BALB/c mice.

### Example 5: The effect of the conditioned media of human HCC cells that express LECT2 on angiogenesis

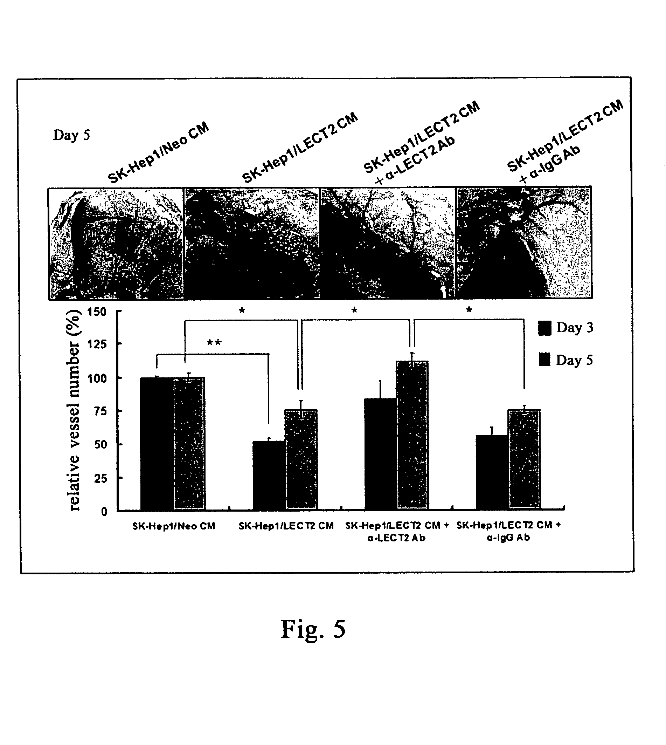

In this example, the effect of the conditioned media with SK-Hep1 cells that overexpress LECT2 on angiogenesis was evaluated using chick chorioallantoic membrane assay (CAM assay). This media were from SK-Hep1 cells transfected with LECT2 or the vector. Cancer cells were cultured in DMEM media without bovine fetal serum for 48 hours. The conditioned media of SK-Hep1 cells transfected with LECT2 and the vector were filtered by low speed centrifugation. CAM assay of chicken embryos is a suitable model for studying vascular response to multiple regulatory factors in the process of *in vivo* angiogenesis. Fertilized eggs were cultured in an incubator with an environment of controlled humidity and aeration at 100°F for 14 days. At Day 9, a small hole was bored on the top of the eggs, above the major vascular region to separate the chick chorioallantoic membrane with the shell membrane. SK-Hep1/Neo conditioned media, SK-Hep1/LECT2 conditioned media, or SK-Hep1/LECT2 conditioned media pretreated with anti-LECT2 or anti-IgG antibody were used for treatment. Conditioned media which have a total volume of 20 µL were dripped to a small disc of 5mm diameter, which covered the chick chorioallantoic membrane (3 chicken embryos treated in each group). The hole on the top of the egg was sealed with air permeable tape, and the eggs were put back into the incubator. At the third day (Day 12) and fifth day (Day 14) after the treatment, the eggs were carefully opened up. Angiogenesis in chick chorioallantoic membrane was evaluated by determing the length and density of the vessels. The effect of the LECT2 recombinant protein on angiogenesis was assayed by comparison between the numbers of the vessels in the control and the experimental groups, which were assayed with Image-Pro Plus 4.5 software. According to Figure 5, the conditioned media with overexpression of LECT2 protein inhibited the angiogenesis of the capillary bed in the chick chorioallantoic membrane. As found in the CAM assay, for the conditioned media with the LECT2 protein overexpression, addition of LECT2 antibody to compete for the binding of LECT2 protein in the conditioned media in advance would improve the angiogenesis when compared to the control group in which a IgG neutralizing antibody was added. Therefore, in the CAM assay, the conditioned media of SK-Hep1 cells overexpressing LECT2 would inhibit angiogenesis.

### Example 6: The effect of the conditioned media of HCC cells which overexpress LECT2 on the formation of tube-like structures

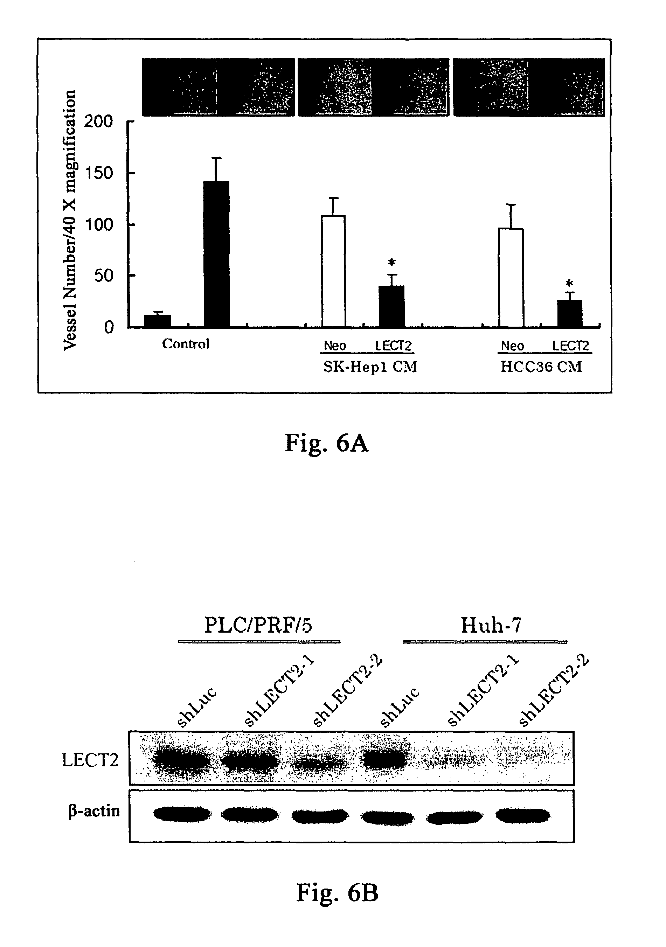

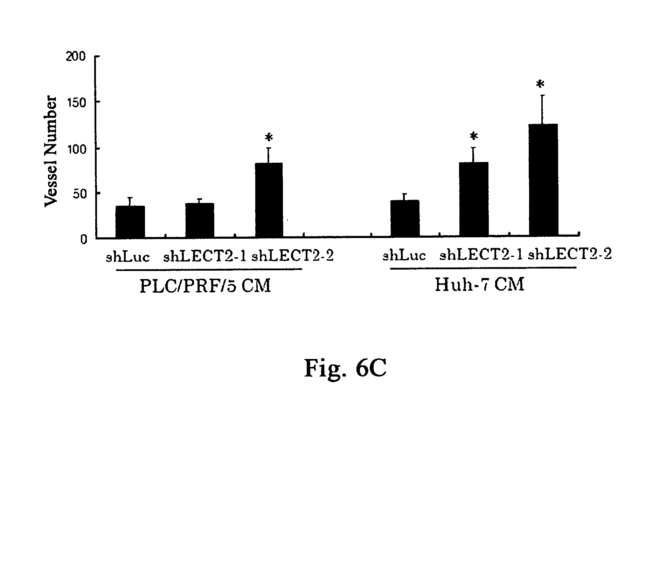

Endothelial cells will align on matrigel within 6 hours and form 2-dimensional microtube-like network *in vitro.* Matrigel assay is an extracellular matrix (ECM) assay which provides a physiological environment useful for analysis of the morphology, biochemical function and gene expression of endothelial cells. Vascular endothelial cells were placed on Matrigel and allowed to differentiate and form microtube-like structures. On Matrigel, formation of microtube-like structures requires cell-matrix interaction, cell-cell communication and cell migration. In order to analyze the *in vitro* effect of the LECT2 recombinant protein on angiogenesis, vascular endothelial cells were placed in a 24-well culture plate covered with Matrigel, treated with the conditioned media of SK-Hep1 or HCC36 cells which were transfected with LECT2 or Neo, respectively, for 6 hours, and observed for formation of tube-like structures using an inverted phase contrast microscope. Under microscopic observation, 6 tube-like structures randomly selected within the visual field were compared to each other. According to Figure 6A, SK-Hep1/LECT2 conditioned media or HCC36/LECT2 conditioned media, rather than the control media (SK-Hep1/Neo conditioned media or HCC/Neo conditioned media) inhibited the formation of tube-like structures on Matrigel layers. As positive controls, Vascular endothelial cells were cultured in 24-well plates covered with Matrigel and treated with VEGF-A for 6 hours. In order to confirm that the LECT2 recombinant protein is an important factor for regulation of angiogenesis in the treatment of angiogenesis, the expressions of PLC/PRF/5 and endogenous LECT2 (shLECT2-1 and shLECT2-2) in Huh-7 cells were further inhibited. According to Figure 6B, the transformants which have endogenous LECT2 inhibited were analysed by using Western blotting. According to Figure 6C, the conditioned media of PLC/PRF/5 and Huh-7 cells which have endogenous LECT2 inhibited improved the tube-like structure forming capacity in vascular endothelial cells. Therefore, it can be known that the conditioned media of cells in which LECT2 is inhibited improved the formation of tube-like structures in vascular endothelial cells.

### Example 7: The effect of the conditioned media of HCC cells overexpressing LECT2 on cell migration

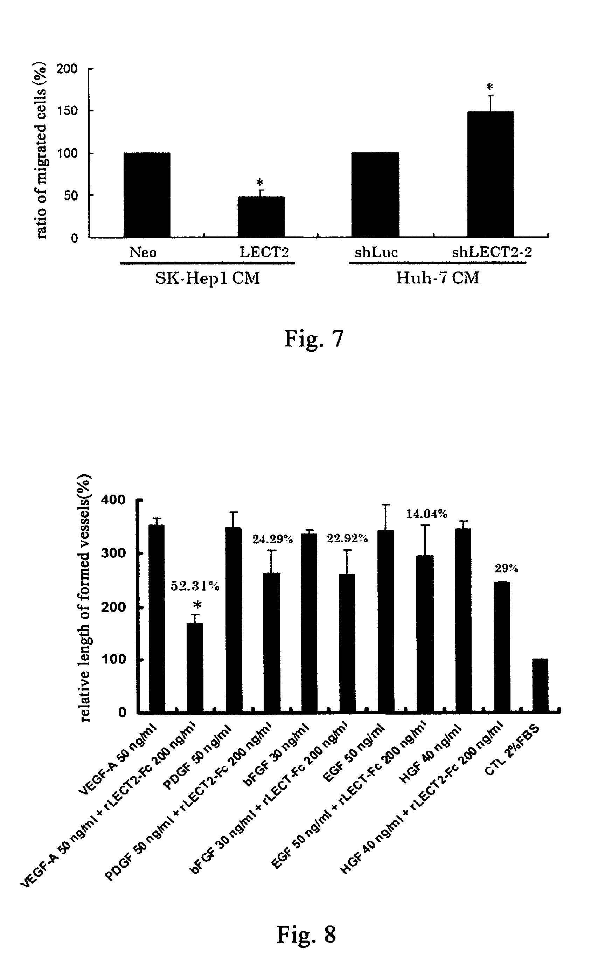

Vascular endothelial cells were cultured in M199 medium in a 24-well culture plate (7x10⁴ cells/well). After 24 hours, medium with 5% fetal bovine serum was exchanged to the monolayer of vascular endothelial cells, and cell-free monolayers were obtained by scratching a straight line with blue pipette tips. The HCC conditioned media containing overexpressed LECT2 (SK-Hep1/LECT2 CM) and the HCC conditioned media of the vector control group (SK-Hep1/Neo CM), or the HCC conditioned media with inhibited endogenous LECT2 (Huh-7/shLECT2-2 CM) and the HCC conditioned media of the vector control group (Huh-7/shLuc CM) were cultured under 370 for 14 hours, followed by observing for the migration ability of endothelial cells using inverted phase contrast microscope, and measuring for the degrees of cell migration under 4 separate randomly selected visual fields. The images were obtained via analysis performed by Image Pro-Plus 4.5 software. For each plate, 4 visual fields were observed, and the scratch width after cell removal was measured. According to Figure 7, vascular endothelia cells moved to the scratch regions 14 hours after cultured in the conditioned media of 50% LECT2 transfected or endogenous LECT2 inhibited. LECT2 conditioned media would inhibit the migration of vascular endothelial cells, and the Huh-7 conditioned media with endogenous LECT2 inhibited (Huh-7/shLECT2-2 CM) would improve the repair response of vascular endothelial cells. Therefore, it can be understood that HCC conditioned media which overexpress LECT2 can inhibit the migration ability of vascular endothelial cells.

### Example 8: The effect of LECT2 recombinant protein on vascular endothelial growth factor induced formation of tube-like structures

Vascular endothelial cells were placed on Matrigel of 24-well plates, treated with VEGF-A (50ng/mL), PDGF (50ng/mL), bFGF (30ng/mL), EGF (50ng/mL), HGF (40ng/mL) or LECT2 recombinant protein (200ng/mL) for 6 hours and assayed for the formation of microtube-like structures. From the results, it can be known that LECT2 recombinant protein effectively inhibited VEGF-A induced neogenesis of vascular tube-like structures, and its inhibition ratio was 52% with respect to the group with VEGF-A (50ng/mL) treatment, as shown in Figure 8. Therefore, LECT2 recombinant protein can inhibit vascular growth factor VEGF-A, PDGF, bFGF, EGF, HGF induced formation of tube-like structures of endothelial cells to different degrees, especially for the VEGF-A.

### Example 9: The effect of LECT2 recombinant protein on VEGF-A induced angiogenesis

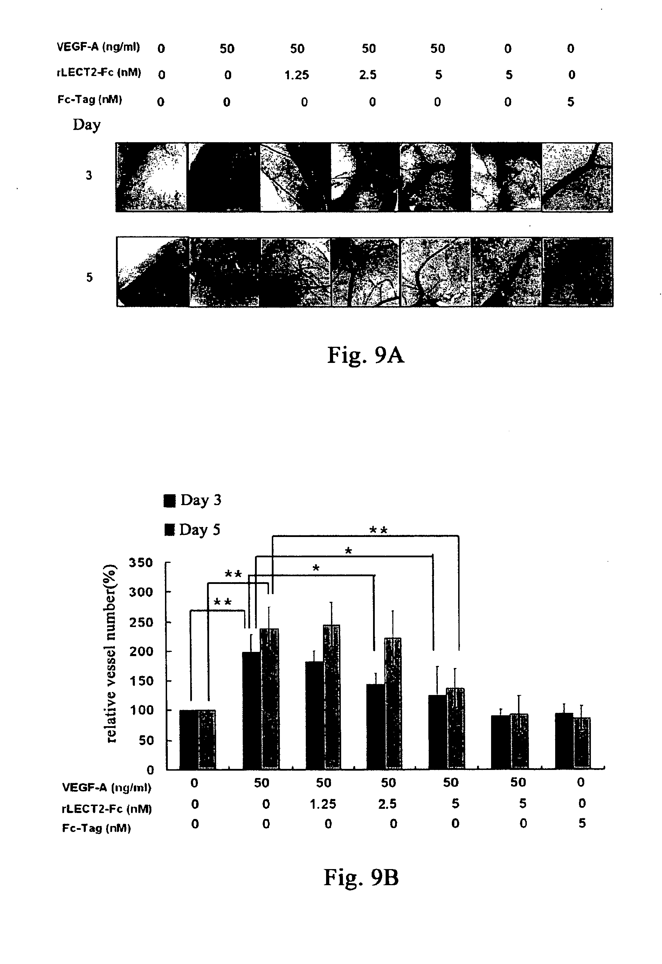

The effect of LECT2 recombinant protein on VEGF-A induced angiogenesis was analyzed by CAM assay. Chick chorioallantoic membranes of 9-day old chicken embryo were treated with VEGF-A and different concentrations of LECT2 recombinant proteins (0, 1.25, 2.5 and 5nM) or Fc-Tag (control) (three chicken embryos were treated in each group): treatments with a total volume of 20µL were dripped onto discs of 5mm diameter, the discs were covered on the chick chorioallantoic membranes. The assay was done at third day (*i.e*., Day 12) or fifth day (*i.e*., Day 14) after treatment of chick chorioallantoic membranes with VEGF-A and LECT2 recombinant protein. According to Figure 9A, VEGF-A (50ng/mL) significantly improved the capillary bed region of chorioallantois compared with control. According to Figure 9B, from CAM assay results, it was known that at Day 12, 2.5nM and 5nM LECT2 recombinant protein could significantly inhibit VEGF-A induced angiogenesis. At Day 14, 5nM LECT2 recombinant protein could inhibit the capillary bed region of chorioallantois induced by VEGF-A. Therefore, it can be known that the LECT2 recombinant protein can inhibit the VEGF-A induced angiogenesis.

### Example 10: The effect of LECT2 recombinant protein on VEGF-A induced in vitro angiogenesis

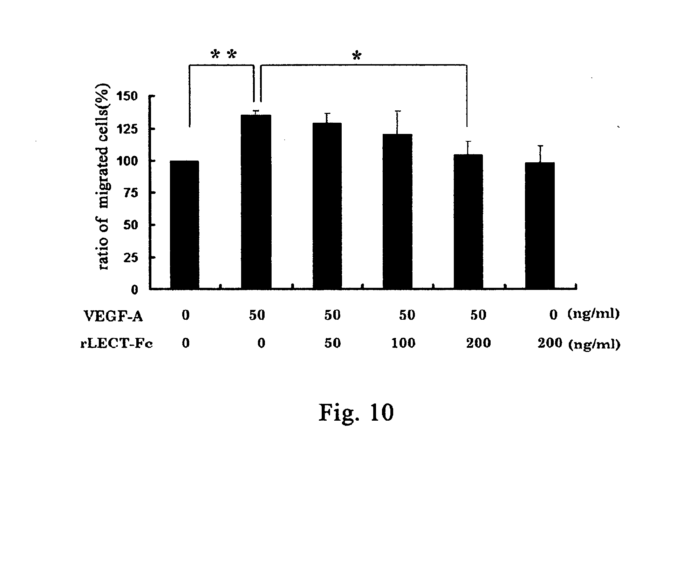

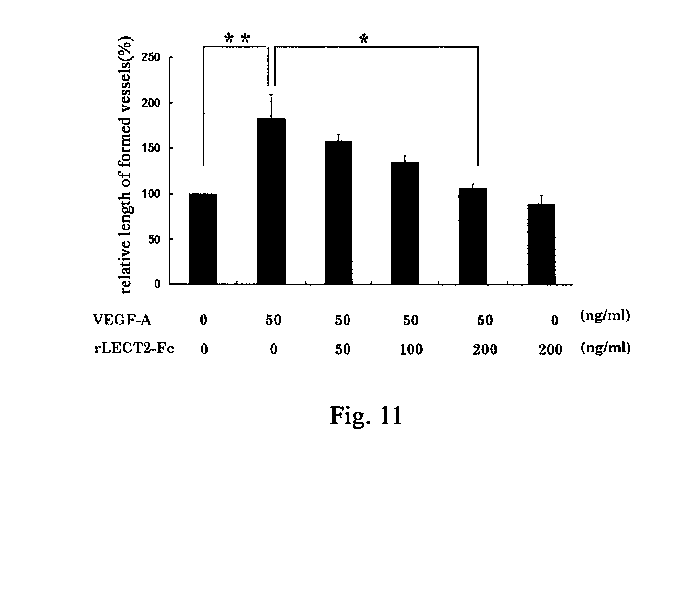

This example analyzed the effect of the LECT2 recombinant protein on vascular endothelial cell migration and angiogenesis. Vascular endothelial cell migration is one of the important factors of angiogenesis, thus the effect of rLECT2 protein on the migration ability of vascular endothelial cell induced by VEGF-A was analyzed. Endothelial cell migration was effected by treatment with 2% FBS (control group), VEGF-A (50ng/mL), VEGF-A (50ng/mL) together with different concentrations of LECT2 recombinant protein (0∼200ng/mL), and LECT2 recombinant protein (200ng/mL) for 14 hours. VEGF-A (50ng/mL) could improve the migration of vascular endothelial cells. From Figure 10, LECT2 recombinant protein inhibited the VEGF-A induced migration in a dose-dependent manner. Furthermore, specifically 200ng/mL dose of LECT2 recombinant protein significantly inhibited the VEGF-A induced migration of vascular endothelial cells. Since LECT2 recombinant protein could inhibit VEGF-A induced cell migration, the effect of LECT2 recombinant protein against angiogenesis during vascular formation was further analyzed. In this analysis, formation of microtube-like structures was assayed with 0.1% BSA (control) or VEGF-A (50ng/mL) combined with different concentrations of LECT2 recombinant protein (0-200ng/mL). From Figure 11, the LECT2 recombinant protein disintegrated VEGF-A induced microtubular network in a dose-dependent manner. Therefore, LECT2 recombinant protein can inhibit VEGF-A induced microtube-like structures of vascular endothelial cells *in vitro.*

### Example 11: The effect of LECT2 recombinant protein on formation of vascular tube-like structures induced by the conditioned media of different cancer cells

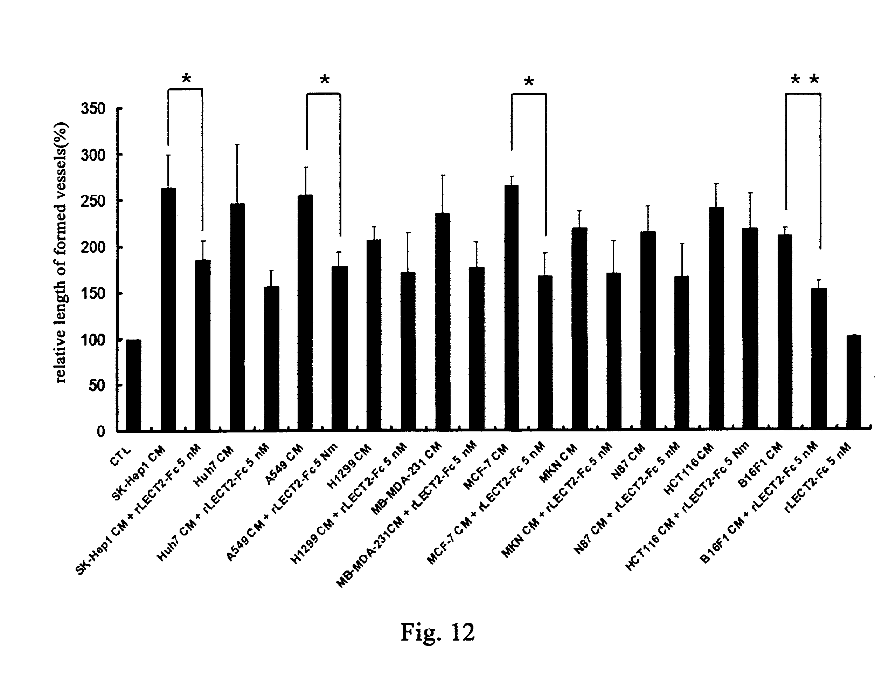

This example analyzed the effect of the LECT2 recombinant protein on angiogenesis of different cancer cell strains. Vascular endothelial cells were placed on Matrigel of 24-well plates, treated with the conditioned media of various cancer cell strains for 6 hours or with the conditioned media of cancer cell strains combined with rLECT2 (5nM) for 6 hours, said cancer cells including liver cancer cells (SK-Hep1 and Huh-7), lung cancer cells (A549 and H1299), breast cancer cells (MB-MDA-231 and MCF7), stomach cancer cells (MKN and N87), rectal cancer cells (HCT 116) and murine melanoma cells (B16F1). Figure 12 shows the results of treatments with cancer cell conditioned media alone or combined with the rLECT2 protein (5nM) of vascular endothelial cells for 6 hours. Therefore, the LECT2 recombinant protein can inhibit the formation of vascular tube-like structures of endothelial cells induced by the conditioned media of SK-Hep1, A549, MCF-7, and B16F1 cells.

### Example 12: The effect of LECT2 protein on tumorigenesis of melanoma and pneunomocyte tumor and angiogenesis

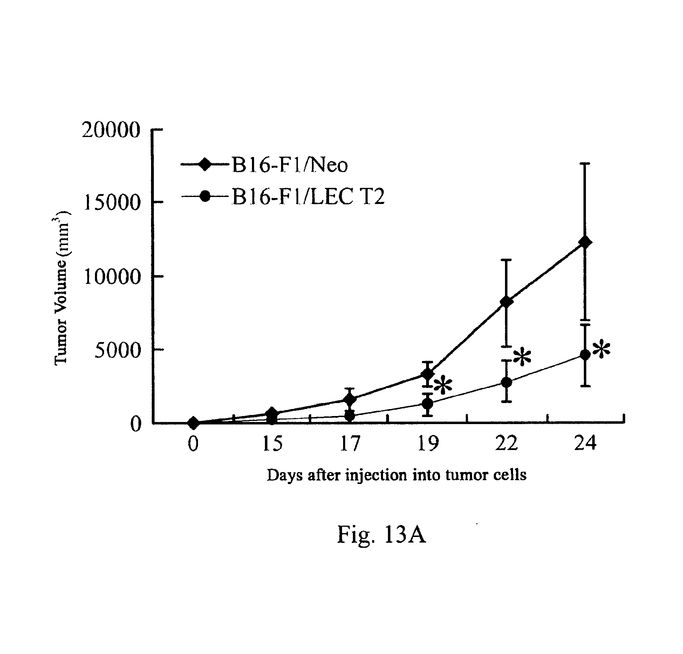

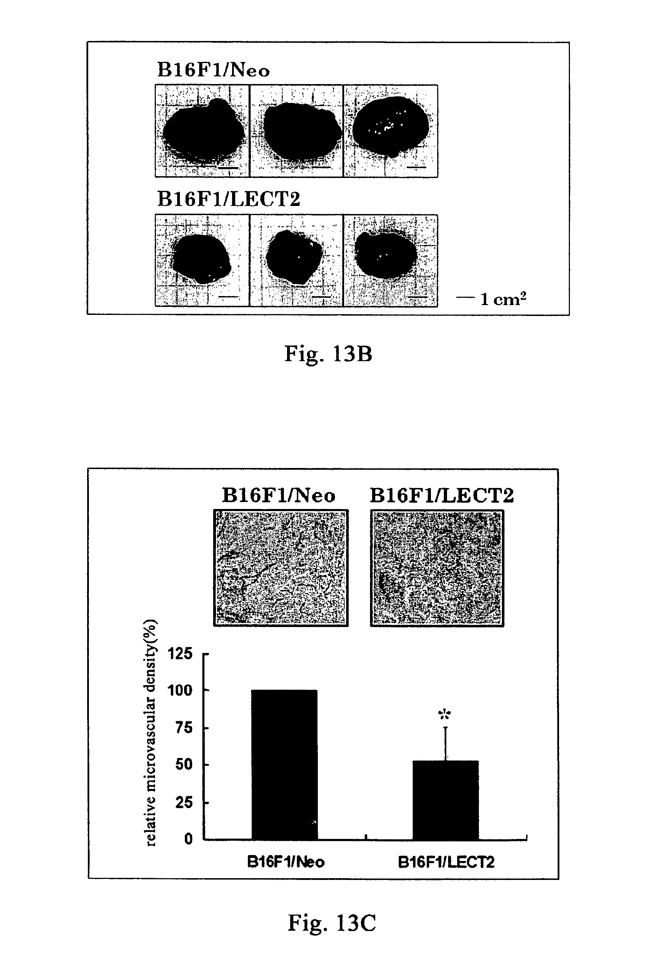

First, murine melanoma cell strain B16F1 which overexpresses LECT2 was constructed. After screening with hygromycin B, the expression level of LECT2 was analyzed, and the effects of LECT2 protein on tumorigenesis and angiogenesis were evaluated using *in vivo* melanoma xenograft model. B16F1 cells (B16F1/Neo and B16F1/LECT2) were injected subcutaneously into 7-week old female C57BU6 mice, with 5x10⁵ cells for each mouse. According to Figure 13A, at Day 18 after the cell injection, the average tumor volume of mice injected with B16F1/Neo was significantly larger than that of mice injected with B16F1/LECT2. At Day 24, mice were sacrificed and subcutaneous tumor taken out and photographed, as shown in Figure 13B. Furthermore, the effect of LECT2 protein overexpressing tumor cells on angiogenesis and vessel number in tumor xenograft was assayed by immunohistochemical staining. According to Figure 13C, tumor samples were fixed with formalin, embedded in paraffin, and the sections were stained via a specific vessel staining method (immunohistochemically staining the endothelial marker CD31). From the statistical results, for the tumors of vector control cells, the vessel number per mm² volume was twice that for tumors of LECT2 transfected cells. Therefore, LECT2 can inhibit the growth of B16F1 melanoma cells and angiogenesis in tumors in C57BL/6J mice.

### Example 13: The effect of LECT2 protein on tumor growth of nude mice

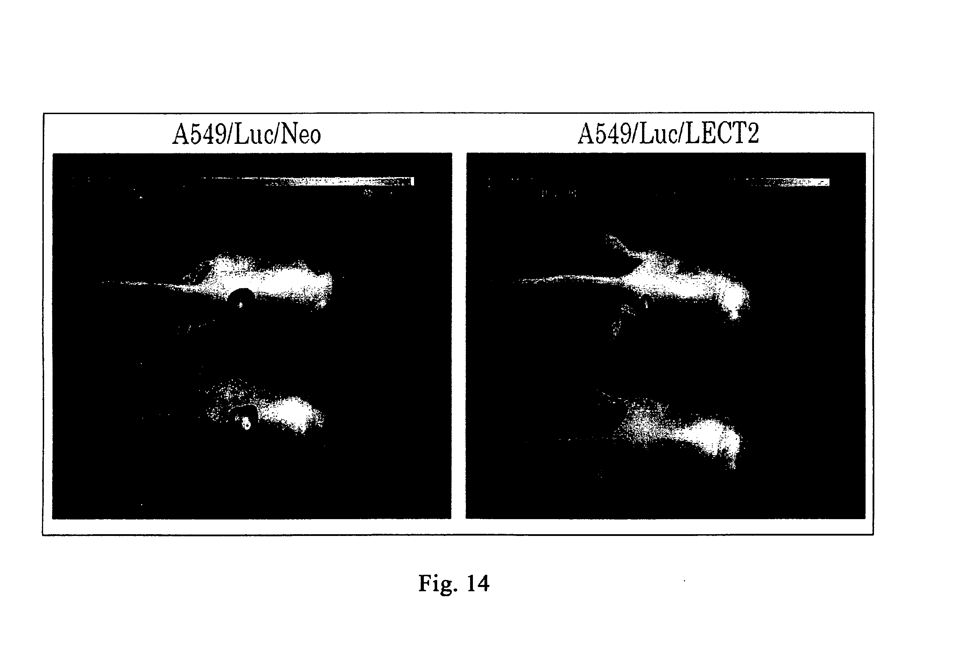

Lung cancer A549 cells were subcutaneously injected into 7-week old female nude mice with each mouse injected with 5x10⁶ cells. At Day 20 after A549 cell injection, D-luciferin (20mg/mL/kg body weight of mouse) was intraperitoneally injected. The mice were anaesthetized and photographed with Berthold/NightOWL imaging system for 5 minutes. According to Figure 14, even with brief exposure (30 seconds), color images showed subcutaneous bright spots in the back of all A549/Luc/Neo tumor mice, although such spots were significantly smaller in A549/Luc/LECT2 tumor mice, indicating that LECT2 could inhibit the growth of lung cancer cells in nude mouse tumors. Therefore, LECT2 can effectively inhibit the tumor growth of A549 cells in nude mice.

While the present invention has been disclosed with respect to the preferred examples above, it shall not be construed as any limitation to the present invention. Any person skilled in the art can make alteration and modification without departing from the spirits and scope of the present invention. Therefore the scope of the present invention is decided by that defined by the attached claims.

## Claims

1. A composition for inhibiting pathologic angiogenesis in an individual, comprising an effective amount of LECT2 protein or analogue thereof, and a pharmaceutically acceptable carrier.

2. The composition for inhibiting pathologic angiogenesis according to Claim 1, wherein the analogue of LECT2 protein is an LECT2 reconbinant protein.

3. The composition for inhibiting pathologic angiogenesis according to Claim 1, wherein the LECT2 protein or analogue thereof is an inhibitor of an angiogenic factor.

4. The composition for inhibiting pathologic angiogenesis according to Claim 3, wherein the angiogenic factor is selected from the group consisting of vascular endothelial growth factor (VEGF), platelet-derived growth factor (PDGF), fibroblast growth factor (FGF), epidermal growth factor (EGF), and hepatocyte growth factor (HGF).

5. The composition for inhibiting pathologic angiogenesis according to Claim 3, wherein the angiogenic factor is a vascular endothelial growth factor.

6. The composition for inhibiting pathologic angiogenesis according to Claim 1, wherein the LECT2 protein or analogue thereof is an inhibitor of endothelial cell migration.

7. The composition for inhibiting pathologic angiogenesis according to Claim 1, wherein the LECT2 protein or analogue thereof is an inhibitor of vascular invasion of tumors.

8. The composition for inhibiting pathologic angiogenesis according to Claim 1, wherein the pathologic angiogenesis is accompanied with a cell proliferative disorder.

9. The composition for inhibiting pathologic angiogenesis according to Claim 8, wherein the cell proliferative disorder is a cancer.

10. The composition for inhibiting pathologic angiogenesis according to Claim 9, wherein the cancer is selected from the group consisting of a liver cancer, a lung cancer, a breast cancer, a colorectal cancer, a stomach cancer and a melanoma.

11. The composition for inhibiting pathologic angiogenesis according to Claim 9, wherein the cancer is a lung cancer or a liver cancer.

12. The composition for inhibiting pathologic angiogenesis according to Claim 1, wherein the individual is a mammal.

13. The composition for inhibiting pathologic angiogenesis according to Claim 1, wherein the individual is a human.

14. A composition for treatment and/or inhibition of cell proliferative disorder in an individual, comprising an effective amount of LECT2 protein or analogue thereof, and a pharmaceutically acceptable carrier.

15. The composition for treatment and/or inhibition of cell proliferative disorder according to Claim 14, wherein the LECT2 protein analogue is an LECT recombinant protein.

16. The composition for treatment and/or inhibition of cell proliferative disorder according to Claim 14, wherein the cell proliferative disorder is a cancer.

17. The composition for treatment and/or inhibition of cell proliferative disorder according to Claim 16, wherein the cancer is selected from the group consisting of a liver cancer, a lung cancer, a breast cancer, a colorectal cancer, a stomach cancer and a melanoma.

18. The composition for treatment and/or inhibition of cell proliferative disorder according to Claim 16, wherein the cancer is a lung cancer or a liver cancer.

19. The composition for treatment and/or inhibition of cell proliferative disorder according to Claim 14, wherein the LECT2 protein or analogue thereof inhibits tumor growth of the individual.

20. The composition for treatment and/or inhibition of cell proliferative disorder according to Claim 14, wherein the LECT2 protein or analogue thereof is an inhibitor of endothelial cell migration of tumors.

21. The composition for treatment and/or inhibition of cell proliferative disorder according to Claim 14, wherein the LECT2 protein or analogue thereof is an inhibitor of of vascular invasion of tumors.

22. The composition for treatment and/or inhibition of cell proliferative disorder according to Claim 14, wherein the individual is a mammal.

23. The composition for treatment and/or inhibition of cell proliferative disorder according to Claim 14, wherein the individual is a human.

24. Use of LECT2 protein or analogue thereof for the preparation of a pharmaceutical composition for inhibiting pathologic angiogenesis.

25. The use of LECT2 protein or analogue thereof according to Claim 24, wherein the LECT2 protein analogue is an LECT2 recombinant protein.

26. Use of LECT2 protein or analogue thereof for the preparation of a pharmaceutical composition for the treatment and/or inhibition of cell proliferative disorder.

27. The use of LECT2 protein or analogue thereof according to Claim 26, wherein the cell proliferative disorder is a cancer.

28. The use of LECT2 protein or analogue thereof according to Claim 27, wherein the cancer is selected from the group consisting of a liver cancer, a lung cancer, a breast cancer, a colorectal cancer, a stomach cancer and a melanoma.

29. The use of LECT2 protein or analogue thereof according to Claim 27, wherein the cancer is a lung cancer or a liver cancer.

30. The use of LECT2 protein or analogue thereof according to Claim 26, wherein the LECT2 protein analogue is an LECT2 recombinant protein.

31. A method for inhibiting pathologic angiogenesis, comprising administering an effective amount of LECT2 protein or analogue thereof, and a pharmaceutically acceptable carrier to an individual.

32. The method for inhibiting pathologic angiogenesis according to Claim 31, wherein the LECT2 protein analogue is an LECT2 recombinant protein.

33. The method for inhibiting pathologic angiogenesis according to Claim 31, wherein the LECT2 protein or analogue thereof inhibits the activity of an angiogenic factor.

34. The method for inhibiting pathologic angiogenesis according to Claim 33, wherein the angiogenic factor is selected from the group consisting of vascular endothelial growth factor (VEGF), platelet-derived growth factor (PDGF), fibroblast growth factor (FGF), epidermal growth factor (EGF), and hepatocyte growth factor (HGF).

35. The method for inhibiting pathologic angiogenesis according to Claim 33, wherein the angiogenic factor is a vascular endothelial growth factor.

36. The method for inhibiting pathologic angiogenesis according to Claim 31, wherein the LECT2 protein or analogue thereof inhibits the migration of endothelial cell.

37. The method for inhibiting pathologic angiogenesis according to Claim 31, wherein the LECT2 protein or analogue thereof inhibits vascular invasion of tumors.

38. The method for inhibiting pathologic angiogenesis according to Claim 31, wherein the pathologic angiogenesis is accompanied with a cell proliferative disorder.

39. The method for inhibiting pathologic angiogenesis according to Claim 38, wherein the cell proliferative disorder is a cancer.

40. The method for inhibiting pathologic angiogenesis according to Claim 39, wherein the cancer is selected from the group consisting of a liver cancer, a lung cancer, a breast cancer, a colorectal cancer, a stomach cancer and a melanoma.

41. The method for inhibiting pathologic angiogenesis according to Claim 39, wherein the cancer is a lung cancer or a liver cancer.

42. The method for inhibiting pathologic angiogenesis according to Claim 31, wherein the individual is a mammal.

43. The method for inhibiting pathologic angiogenesis according to Claim 31, wherein the individual is a human.
